Europäisches Patentamt

European Patent Office

Office européen des brevets

(19)

(11) Veröffentlichungsnummer: **0 218 204 B1**

(12) **EUROPÄISCHE PATENTSCHRIFT**

(45) Veröffentlichungstag der Patentschrift: **11.12.91**

(51) Int. Cl.⁵: **C12N 15/76**, C12N 1/21, //(C12N1/21,C12R1:465)

(21) Anmeldenummer: **86113627.3**

(22) Anmeldetag: **02.10.86**

(54) Promotor-Anordnung für Streptomyceten-Vektoren.

(30) Priorität: **10.10.85 DE 3536182**

(43) Veröffentlichungstag der Anmeldung:
**15.04.87 Patentblatt 87/16**

(45) Bekanntmachung des Hinweises auf die
Patenterteilung:
**11.12.91 Patentblatt 91/50**

(84) Benannte Vertragsstaaten:
**AT BE CH DE ES FR GB GR IT LI LU NL SE**

(56) Entgegenhaltungen:
**EP-A- 0 105 521**
**EP-A- 0 152 830**
**EP-A- 0 187 630**

**GENE, Band 28, April 1984, Seiten 83-91, El-sevier Science Publishers, Amsterdam, NL;
B. JAURIN et al.: "Streptomyces lividans RNA polymerase recognizes and uses Escheri-chia coli transcriptional signals"**

(73) Patentinhaber: **HOECHST AKTIENGESELL-SCHAFT**
**Postfach 80 03 20**
**W-6230 Frankfurt am Main 80(DE)**

(72) Erfinder: **Koller, Klaus-Peter, Dr.**
**Carl-Orff-Weg 12**
**W-6232 Bad Soden am Taunus(DE)**
Erfinder: **Riess, Günther Johannes, Dr.**
**Loreleistrasse 59**
**W-6230 Frankfurt am Main 80(DE)**

**Beschreibung**

Die Erfindung betrifft eine Kombination von Promotoren, die in Streptomyceten wirksam und hintereinander angeordnet sind. Durch diese "Tandem-Anordnung" wird eine erhebliche Steigerung der Proteinexpression bewirkt. Die Erfindung bezieht sich weiterhin auf Vektoren, die diese Promotorenanordnung enthalten, Streptomyceten-Wirtsstämme, die solche Vektoren enthalten und ihre Verwendung zur Herstellung von Proteinen. Bevorzugte Ausgestaltungen der Erfindung werden im folgenden näher erläutert bzw. in den Patentansprüchen definiert.

Über die DNA-Struktur von Promotoren, die in Streptomyceten wirksam sind, ist noch verhältnismäßig wenig bekannt. Es wurde nun gefunden, daß durch eine Anordnung von zwei Streptomyceten-Promotoren nacheinander die Proteinexpression in unerwartetem Ausmaß gesteigert werden kann. Hierbei müssen die beiden Promotoren nicht unmittelbar hintereinander angeordnet sein, was auch bei den heutigen, noch geringen Kenntnissen hinsichtlich der DNA-Struktur dieser Promotoren mit Schwierigkeiten verbunden wäre. Es genügt also, wenn die Promotoren in Tandem-Anordnung zueiner und wirksam zu dem zu exprimierenden Strukturgen angeordnet sind, wobei zwischen den beiden Promotoren ein mehr oder weniger langer DNA-Abschnitt angeordnet sein kann. Die günstigste Dimension dieser DNA-Brücke kann durch einfache Vorversuche leicht ermittelt werden, wobei man beispielsweise chemisch synthetisierte DNA-Brücken in Form geeigneter Linker bzw. Adaptoren einsetzen kann.

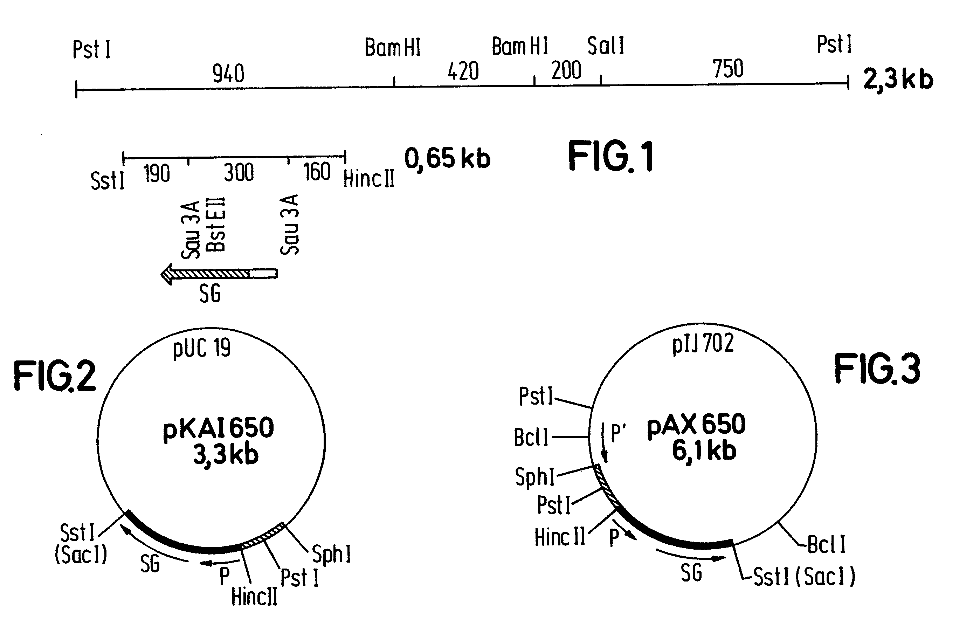

Ein geeigneter in Streptomyceten wirksamer Promotor kann aus dem Hybridplasmid pKAI 1 gewonnen werden, das in der Deutschen Offenlegungsschrift 3 331 860 beschrieben ist. Hierzu wird der 2,3 kb-DNA-Abschnitt, der in der Figur 2 dieser Offenlegungsschrift näher erläutert ist, mit den Restriktionsenzymen Sst I und Hinc II umgesetzt und das 650 bp umfassende Fragment isoliert. Dieses DNA-Fragment enthält das Strukturgen für den α-Amylase-Inhibitor "Tendamistat" und den dazugehörigen Promotor. Diese DNA-Sequenz liegt innerhalb des 940 bp umfassenden Abschnitts zwischen den Restriktionsstellen für die Enzyme Pst I und Bam HI (Figur 2 der Deutschen Offenlegungsschrift 3 331 860).

Ein weiterer Promotor, der für die erfindungsgemäße "Tandem-Anordnung" geeignet ist, findet sich in dem handelsüblichen Plasmid pIJ 702 (E. Katz et al., J. Gen. Microbiol. 129 (1983) 2703 - 2714; D.A. Hopwood et al., Genetic Manipulations of Streptomyces, A Laboratory Manual, The John Innes Foundation, Norwich, England, 1985, Seite 292). Hierzu wird dieses Plasmid mit den Restriktionsenzymen Sph I und Pst I geschnitten und das 350 bp umfassende Fragment isoliert. Es ist auch möglich, das Plasmid mit den Restriktionsenzymen Sph I und Bcl I umzusetzen und das 270 bp umfassende DNAFragment zu isolieren. Auf diesem 270 bp DNA-Fragment befindet sich der Promotor für das mel-Gen, das für das Protein Tyrosinase kodiert.

Diese DNA-Fragmente mit dem mel-Promotor können dann - im korrekten Leserahmen - in einen beliebigen Expressionsvektor in der Weise integriert werden, daß sie vor einem anderen in Streptomyceten wirksamen Promotor oder zwischen diesem Promotor und dem Strukturgen liegen.

In einer anderen Ausgestaltung der Erfindung wird nicht der mel-Promotor aus dem Plasmid pIJ 702 isoliert, sondern in dieses Plasmid das Strukturgen und der zweite Promotor - wieder in Tandem-Anordnung - integriert. Es ist zweckmäßig, den zweiten Promotor und das Strukturgen als Einheit hinter den mel-Promotor einzubauen, also beispielsweise von dem genannten DNA-Fragment mit dem Tendamistat-Strukturgen und dem dazugehörigen Promotor Gebrauch zu machen. Diese Genstruktur führt zu einer um etwa den Faktor 10 erhöhten, korrekten Expression und Sekretion des α-Amylase-Inhibitors Tendamistat.

Beim Einsatz von Plasmiden hoher Kopienzahl pro Zelle kann bei langer Fermentationsdauer die Expression von Proteinen zurückgehen. In solchen Fällen empfiehlt sich die Verwendung von Vektoren mit niedriger Kopienzahl.

Entsprechend dieser hier nur beispielhaft wiedergegebenen Genstruktur können auch andere in Streptomyceten wirksame Promotoren in solche "Tandem-Anordnungen" eingebracht werden und beliebige Strukturgene exprimiert werden.

In den folgenden Beispielen wird die Erfindung näher erläutert.

Beispiel 1

Aus 7 μg DNA aus dem Plasmid pKAI 1 (Deutsche Offenlegungsschrift 3 331 860) wird ein 650 bp Hinc II-Sst I-Fragment durch Elektroelution isoliert. Die Anordnung dieses Fragments innerhalb des 2,3 kb-Fragments aus dem Plasmid pKAI 1 ist durch die Figur 1 charakterisiert, in der "SG" das Strukturgen für den α-Amylase-Inhibitor bezeichnet. Das 650 bp-Fragment enthält zusätzlich zu dem Strukturgen alle für die Expression in Streptomyces lividans TK 24 notwendigen regulatorischen Bereiche.

Der handelsübliche E. coli-Vektor pUC 19 (c. Yanisch-Perron et al., Gene 33 (1985), 103 - 119; New England Biolabs 1985/86 Catalog, S. 90/91; BRL, Bethesda Research Laboratories Catalogue & Reference Guide, 1985, S. 136/137) wird mit den Restriktionsendonukleasen Hinc II und Sst I doppelt verdaut und das linearisierte Plasmid mit dem vorstehend genannten 650 bp Sst I-Hinc II-Fragment mit dem Tendamistatgen ligiert. Nach Transformation von E. coli JM 101 werden Klone isoliert, die das rekombinante Plasmid mit der Bezeichnung pKAI 650 enthalten, welches das 650 bp-Fragment eingebaut enthält. Dieses Plasmid ist in der Figur 2 (nicht maßstabsgerecht) gezeigt, in der P die Promotorregion bezeichnet.

Isolierte pKAI 650-Plasmid-DNA wird nach Doppelverdauung mit den Restriktionsendonukleasen Sst I und Sph I bzw. mit Sst I und Pst I einer präparativen Gelelektrophorese und Elektroelution unterzogen und das ca. 650 bp umfassende Fragment isoliert.

## Beispiel 2

Das Plasmid pIJ 702 (erhältlich von der John Innes Foundation, Norwich, England) wird mit den Restriktionsendonukleasen Sst I und Sph I verdaut und das 400 bp Sst I-Sph I-Fragment von der verbleibenden Vektor-DNA durch Agarosegel-Elektrophorese abgetrennt. 1 µg der isolierten, gereinigten Vektor-DNA wird mit 0,3 µg des 650 bp-Sst I-Sph I-Fragments aus pKAI 650 (Beispiel 1) ligiert und mit dem Ligationsprodukt Streptomyces lividans TK 24 (erhältlich bei John Innes Foundation) in an sich bekannter Weise transformiert. Die Selektion der gewünschten Klone erfolgt auf Resistenz gegenüber Thiostrepton und Produktion des $\alpha$-Amylase-Inhibitors gemäß dem folgenden Plattentest:

Die Kolonien werden mit 5 ml einer 0,4 bis 1,0 mg/ml Pankreatin enthaltenden wäßrigen Lösung übergossen und 1 Stunde bei 37°C inkubiert. Die Lösung wird dann entfernt und durch 5 ml eines 2 Gew.-%igen Stärke-Agars ersetzt. Nach 2 Stunden Inkubation bei 37°C werden die Platten mit 5 ml einer Jod-Kaliumjodid-Lösung zur Entwicklung übergossen. Kolonien mit einem blauen Hof zeigen an, daß die Klone Tendamistat synthetisieren und exkretieren.

Das rekombinante Plasmid mit der Bezeichnung pAX 650, das die Synthese des $\alpha$-Amylase-Inhibitors Tendamistat bewirkt, ist in der Figur 3 (nicht maßstabsgerecht) dargestellt. P' bedeutet hierin die Promotorregion des mel-Gens aus pIJ 702.

## Beispiel 3 (Vergleichsbeispiel)

Man verfährt gemäß Beispiel 2, eliminiert jedoch aus dem Plasmid pIJ 702 das 650 bp umfassende Pst I-Sst I-Fragment und ligiert das Restplasmid mit dem etwa 650 bp umfassenden Sst I-Pst I-Fragment aus pKAI 650. Das so erhaltene rekombinante Plasmid pAX 651 ergibt in dem Test auf $\alpha$-Amylase-Inhibierung einen etwa 10fach niedrigeren Wert als mit dem Plasmid pAX 650 (Beispiel 2).

## Beispiel 4

Der "Pendelvektor" pSW1 (Europäische Patentanmeldung mit der Veröffentlichungsnummer 0 158 201, Fig. 26) wird partiell mit dem Restriktionsenzym Bcl I verdaut und die linearen Fragmente von 16,6 kb Länge nach Elektrophorese in einem 0,4%igen Agarosegel isoliert. Die isolierten DNA-Fragmente werden mit gepufferter Phenollösung extrahiert und mit Ethanol gefällt.

Die so gereinigten DNA-Fragmente werden mit dem 1,81 kb Bcl I-Fragment aus pAX650, das das komplette $\alpha$-Amylase-Inhibitorgenträgt, ligiert. Das Ligationsgemisch wird nach S. lividans TK24 transformiert. Rekombinante, Thiostreptonresistente Klone produzieren und sezernieren den $\alpha$-Amylase-Inhibitor Tendamistat auch über lange Fermentationszeiten in konstanter Ausbeute.

## Patentansprüche
Patentansprüche für folgende Vertragsstaaten:
BE, CH, DE, FR, GB, GR, IT, LI, LU, NL, SE

1. In Streptomyceten aktive Promotorenanordnung, dadurch gekennzeichnet, daß ein in Streptomyceten aktiver Promotor, im Tandem hinter einem weiteren in Streptomyceten aktiven Promotor angeordnet ist.

2. Promotorenanordnung nach Anspruch 1, dadurch gekennzeichnet, daß einer der beiden Promotoren der aus dem 350 bp Pst I-Sph I-Fragment des Plasmids pIJ 702 ist.

3. Promotorenanordnung nach Anspruch 2, dadurch gekennzeichnet, daß einer der beiden Promotoren der aus dem 270 bp Bcl I-Sph I-Fragment des Plasmids pIJ 702 ist.

4. Promotorenanordnung nach Anspruch 1, 2 oder 3, dadurch gekennzeichnet, daß einer der Promotoren der aus dem 650 bp Hinc II-Sst I-Fragment innerhalb des 0,94 kb Pst I-BamH I-Fragments des Plasmids pKAI 1 ist.

5. In Streptomyceten aktiver Vektor, gekennzeichnet durch eine Promotorenanordnung nach einem oder mehreren der vorhergehenden Ansprüche.

6. Wirtszelle der Gattung Streptomyces, gekennzeichnet durch einen Gehalt an einem Vektor nach Anspruch 5.

7. Wirtszelle nach Anspruch 6, dadurch gekennzeichnet, daß sie von der Art S. lividans ist.

8. Verfahren zur Herstellung eines Proteins, dadurch gekennzeichnet, daß man in einer Wirtszelle der Gattung Streptomyces einen Vektor zur Expression bringt, der im korrekten Leserahmen eine Promotorenanordnung nach einem oder mehreren der Ansprüche 1 bis 4 enthält.

9. Verfahren nach Anspruch 8, dadurch gekennzeichnet, daß die Wirtszelle von der Art S. lividans ist.

**Patentansprüche für folgende Vertragsstaaten: AT, ES**

1. Verfahren zur Herstellung einer in Streptomyceten aktiven Promotorenanordnung, dadurch gekennzeichnet daß man einen in Streptomyceten aktiven Promotor in Tandem-Anordnung hinter einem weiteren in Streptomyceten aktiven Promotor einbaut.

2. Verfahren nach Anspruch 1, dadurch gekennzeichnet, daß einer der beiden Promotoren der aus dem 350 bp Pst I-Sph I-Fragment des Plasmids pIJ 702 ist.

3. Verfahren nach Anspruch 1, dadurch gekennzeichnet, daß einer der beiden Promotoren der aus dem 270 bp Bcl I-Sph I-Fragment des Plasmids pIJ 702 ist.

4. Verfahren nach Anspruch 1, 2 oder 3, dadurch gekennzeichnet, daß einer der Promotoren der aus dem 650 bp Hinc II-Sst I-Fragment innerhalb des 0,94 kb Pst I-Bam HI-Fragments des Plasmids pKAI 1 ist.

5. Verfahren zur Herstellung eines in Streptomyceten aktiven Vektors, dadurch gekennzeichnet, daß man eine nach einem oder mehreren der vorhergehenden Ansprüche erhaltene Promotorenanordnung in den Vektor einbaut.

6. Verfahren zur Herstellung einer Wirtszelle der Gattung Streptomyces, dadurch gekennzeichnet, daß man die Zelle mit einem nach Anspruch 5 erhaltenen Vektor transformiert.

7. Verfahren nach Anspruch 6, dadurch gekennzeichnet, daß die Wirtszelle von der Art S. lividans ist.

8. Verfahren zur Herstellung eines Proteins, dadurch gekennzeichnet, daß man in einer Wirtszelle der Gattung Streptomyces ein Gen zur Expression bringt, das in einem Vektor eingebaut ist, der eine nach einem oder mehreren der Ansprüche 1 bis 4 erhaltene Promotorenanordnung enthält.

9. Verfahren nach Anspruch 8, dadurch gekennzeichnet, daß die Wirtszelle von der Art S. lividans ist.

**Claims**
**Claims for the following Contracting States: BE, CH, DE, FR, GB, GR, IT, LI, LU, NL, SE**

1. A promoter arrangement active in Streptomycetes, wherein a promoter active in Streptomycetes is arranged in tandem behind another promoter active in Streptomycetes.

2. The promoter arrangement as claimed in claim 1, wherein one of the two promoters is that from the 350 bp Pst I-Sph I fragment of the plasmid pIJ 702.

3. The promoter arrangement as claimed in claim 2, wherein one of the two promoters is that from the 270 bp Bcl I-Sph I fragment of the plasmid pIJ 702.

4. The promoter arrangement as claimed in claim 1, 2 or 3, wherein one of the promoters is that from the 650 bp Hinc II-Sst I fragment within the 0.94 kb Pst I-BamH I fragment of the plasmid pKAI 1.

5. A vector active in Streptomycetes, having a promoter arrangement as claimed in one or more of the preceding claims.

6. A host cell of the genus Streptomyces containing a vector as claimed in claim 5.

7. The host cell as claimed in claim 6, which is of the species S. lividans.

8. A process for the preparation of a protein, which comprises bringing about the expression of a vector in a host cell of the genus Streptomyces, which vector contains, in the correct reading frame, a promoter arrangement as claimed in one or more of claims 1 to 4.

9. The process as claimed in claim 8, wherein the host cell is of the species S. lividans.

**Claims for the following Contracting States: AT, ES**

1. A process for the preparation of a promoter arrangement active in Streptomycetes, which comprises incorporating a promoter active in Streptomycetes in tandem arrangement behind another promoter active in Streptomycetes.

2. The process as claimed in claim 1, wherein one of the two promoters is that from the 350 bp Pst I-Sph I fragment of the plasmid pIJ 702.

3. The process as claimed in claim 1, wherein one of the two promoters is that from the 270 bp Bcl I-Sph I fragment of the plasmid pIJ 702.

4. The process as claimed in claim 1, 2 or 3, wherein one of the promoters is that from the 650 bp Hinc II-Sst I fragment within the 0.94 kb Pst I-Bam HI fragment of the plasmid pKAI 1.

5. A process for the preparation of a vector active in Streptomycetes, which comprises incorporating in the vector a promoter arrangement obtained as claimed in one or more of the preceding claims.

6. A process for the preparation of a host cell of the genus Streptomyces, which comprises transforming the cell with a vector obtained as claimed in claim 5.

7. The process as claimed in claim 6, wherein the host cell is of the species S. lividans.

8. A process for the preparation of a protein, which comprises bringing about the expression of a gene which is incorporated in a vector, in a host cell of the genus Streptomyces, which vector contains a promoter arrangement obtained as claimed in one or more of claims 1 to 4.

9. The process as claimed in claim 8, wherein the host cell is of the species S. lividans.

**Revendications**
**Revendications pour les Etats contractants suivants: BE, CH, DE, FR, GB, GR, IT, LI, LU, NL, SE**

1. Disposition de promoteurs active dans les streptomycètes, caractérisée en ce qu'un promoteur actif dans les streptomycètes est placé en tandem derrière un autre promoteur actif dans les streptomycètes.

2. Disposition de promoteurs selon la revendication 1, caractérisée en ce que l'un des deux promoteurs est le promoteur issu du fragment Pst I-Sph I de 350 bp du plasmide pIJ 702.

3. Disposition de promoteurs selon la revendication 2, caractérisée en ce que l'un des deux promoteurs est le promoteur issu du fragment Bcl I-Sph I de 270 bp du plasmide pIJ 702.

4. Disposition de promoteurs selon la revendication 1, 2 ou 3, caractérisée en ce que l'un des promoteurs est le promoteur issu du fragment Hinc II-Sst I de 650 bp qui est compris dans le fragment Pst I-BamH I de 0,94 kb du plasmide pKAI 1.

5. Vecteur actif dans les streptomycètes, caractérisé par une disposition de promoteurs selon l'une ou plusieurs des revendications précédentes.

6. Cellule hôte du genre Streptomyces, caractérisée par le fait qu'elle contient un vecteur selon la revendication 5.

7. Cellule hôte selon la revendication 6, caractérisée en ce qu'elle est de l'espèce S. lividans.

8. Procédé pour préparer une protéine, caractérisé en ce que l'on apporte, dans une cellule hôte du genre Streptomyces, un vecteur d'expression qui contient une disposition de promoteurs dans le cadre de lecture correct, selon l'une ou plusieurs des revendications 1 à 4.

9. Procédé selon la revendication 8, caractérisé en ce que la cellule hôte est de l'espèce S. lividans.

**Revendications pour les Etats contractants suivants: AT, ES**

1. Procédé pour préparer une disposition de promoteurs active dans les streptomycètes, caractérisé en ce que l'on introduit un promoteur actif dans les streptomycètes dans une disposition en tandem derrière un autre promoteur actif dans les streptomycètes.

2. Procédé selon la revendication 1, caractérisé en ce que l'un des deux promoteurs est le promoteur issu du fragment Pst I-Sph I de 350 bp du plasmide pIJ 702.

3. Procédé selon la revendication 1, caractérisé en ce que l'un des deux promoteurs est le promoteur issu du fragment Bcl I-Sph I de 270 bp du plasmide pIJ 702.

4. Procédé selon la revendication 1, 2 ou 3, caractérisé en ce que l'un des promoteurs est le promoteur issu du fragment Hinc II-Sst I de 650 bp qui est compris dans le fragment Pst I-BamH I de 0,94 kb du plasmide pKAI 1.

5. Procédé pour préparer un vecteur actif dans les streptomycètes, caractérisé en ce que l'on introduit dans le vecteur une disposition de promoteurs obtenue selon l'une ou plusieurs des revendications précédentes.

6. Procédé pour préparer une cellule hôte du genre Streptomyces, caractérisé en ce que l'on transforme la cellule avec un vecteur obtenu selon la revendication 5.

7. Procédé selon la revendication 6, caractérisé en ce que la cellule hôte est de l'espèce S. lividans.

8. Procédé pour préparer une protéine, caractérisé en ce que l'on apporte, dans une cellule hôte du genre Streptomyces, un gène d'expression qui est compris dans un vecteur contenant une disposition de promoteurs selon l'une ou plusieurs des revendications 1 à 4.

9. Procédé selon la revendication 8, caractérisé en ce que la cellule hôte est de l'espèce S. lividans.

FIG.1

FIG.2

FIG.3

EP 0 218 204 B1